# EUROPÄISCHE PATENTSCHRIFT

(11) **EP 4 584 587 B1**
(45) Veröffentlichungstag und Bekanntmachung des Hinweises auf die Patenterteilung: **15.07.2026**
(21) Anmeldenummer: 23768205.9
(22) Anmeldetag: 06.09.2023
(51) Int. Cl.: G01N 33/00, G01N 25/00, G01N 33/22

(54) **VERFAHREN ZUR DIAGNOSE EINES WASSERSTOFFSENSORS, WASSERSTOFFSENSOR, COMPUTERPROGRAMM UND COMPUTERLESBARES MEDIUM**
METHOD FOR DIAGNOSING A HYDROGEN SENSOR, HYDROGEN SENSOR, COMPUTER PROGRAM, AND COMPUTER-READABLE MEDIUM
PROCÉDÉ DE DIAGNOSTIC D'UN CAPTEUR D'HYDROGÈNE, CAPTEUR D'HYDROGÈNE, PROGRAMME INFORMATIQUE ET SUPPORT LISIBLE PAR ORDINATEUR

(30) Priorität: 09.09.2022 DE 102022209399
(43) Veröffentlichungstag der Anmeldung: 16.07.2025
(73) Patentinhaber: Schaeffler Technologies AG & Co. KG, 91074 Herzogenaurach (DE)
(72) Erfinder: KNITTEL, Thorsten, 80687 München (DE); LAUERER, Wolfgang, 80687 München (DE); MEYER, Andreas, 80687 München (DE); PESAHL, Stefan, 80687 München (DE)
(74) Vertreter: Schaeffler Technologies
(86) Internationale Anmeldenummer: PCT/EP2023/074376
(87) Internationale Veröffentlichungsnummer: WO 2024/052371

(56) Entgegenhaltungen:
- US-A1- 2005 155 405
- US-A1- 2011 302 993
- LI JIN ET AL: "The Design and Implementation of Hydrogen Sensor Fault Detection Device", 2021 19TH INTERNATIONAL CONFERENCE ON OPTICAL COMMUNICATIONS AND NETWORKS (ICOCN), IEEE, 23 August 2021 (2021-08-23), pages 1 - 3, XP033991247, DOI: 10.1109/ICOCN53177.2021.9563861

## Beschreibung

### Technisches Gebiet

Die Erfindung betrifft ein Verfahren zur Diagnose eines Wasserstoffsensors, einen Wasserstoffsensor, ein Computerprogramm und ein computerlesbares Medium.

### Stand der Technik

Wasserstoffsensoren zur Ermittlung einer Wasserstoffkonzentration eines Gases weisen in der Regel einen Messraum auf, der Schutz vor der Strömung des Gases bietet. Ein Zugang zu diesem Messraum ist in der Regel durch eine gasdurchlässige und flüssigkeitsundurchlässige Membran verschlossen, um den Eintritt von Flüssigkeit oder Fremdkörpern in den Messraum zu verhindern. Unerwünschterweise kommt es an dieser Membran zu Ablagerungen von Fremdkörpern oder einer Ansammlung einer Flüssigkeit, wodurch eine Messung in der Wasserstoffkonzentration des Gases verfälscht wird.

Die Druckschrift US 9 702 836 B2 betrifft einen Sensor mit einer Fehlererkennungsfunktion.

Weiteren Stand der Technik bilden die US 2005/0 155 405A1, US 2011 / 0 302 993 A1 und Li Jin et al: "The Design and Implementation of Hydrogen Sensor Fault Detection Device", 2012, 19th International Conference on optical Communications and Networks (ICOCN), IEEE, 23. August 2021, Seiten 1 - 3, XP033991247, DOI: 10.1109/ICOCN52177.2021.9563861 bekannt.

### Darstellung der Erfindung, Aufgabe, Lösung, Vorteile

Es ist die Aufgabe der vorliegenden Erfindung ein alternatives Verfahren zu schaffen, welches sich insbesondere durch seine einfache Implementierbarkeit auszeichnet.

Die erste Aufgabe wird durch ein Verfahren mit den Merkmalen von Anspruch 1 gelöst.

Ein Ausführungsbeispiel der Erfindung betrifft ein Verfahren zur Diagnose eines Wasserstoffsensors,
wobei der Wasserstoffsensor einen Messraum, einen Heizer und einen Temperaturfühler umfasst, wobei der Messraum mittels einer Messraumöffnung fluidisch mit der Umgebung des Wasserstoffsensors verbunden ist,
wobei die Messraumöffnung mittels einer Membran verschlossen ist,
wobei die Membran gasdurchlässig und flüssigkeitsundurchlässig ist,
umfassend die Schritte:
   - Temperaturänderung im Messraums mittels des Heizers,
   - Ermittlung eines Diagnosewerts im Messraum mittels des Temperaturfühlers,
   - Feststellung einer Diagnose in Abhängigkeit des Diagnosewerts.

Auf diese Weise kann eine Diagnose des Wasserstoffsensors durchführt werden, ohne zusätzliche Sensorelemente vorsehen zu müssen, da bereits vorhandene Mittel zur Ermittlung der Wasserstoffkonzentration in einem Gas mittels des Wasserstoffsensors zur Diagnose genutzt werden.

Bei der Feststellung der Diagnose handelt es sich bevorzugterweise um die Feststellung der Diagnose des Wasserstoffsensors, besonders bevorzugt seiner Membran und/oder seiner Messraumöffnung.

Besonders vorteilhaft ist es, wenn der Heizer und der Temperaturfühler im Messraum angeordnet sind. Dies ermöglicht eine schnellere Aufheizung und eine genauere Messung.

Auch ist es vorteilhaft, wenn es sich bei dem Wasserstoffsensor um einen, insbesondere thermischen, Sensor zu Ermittlung einer Wasserstoffkonzentration in einem Gas handelt.

Vorzugsweise handelt es sich bei dem Wasserstoffsensor um einen thermischen Sensor, der sich die im Vergleich zu anderen Bestandteilen im zu messenden Gas deutlich höhere Wärmekapazität von Wasserstoff zu Nutze macht, um die Wasserstoffkonzentration in dem zu messenden Gas zu ermitteln.

Auch ist es zu bevorzugen, wenn der Wasserstoffsensor ein Sensorgehäuse umfasst, das insbesondere den Messraum begrenzt und/oder die Messraumöffnung, vorzugsweise an der Oberfläche des Sensorgehäuses, umfasst.

Ferner ist es von Vorteil, wenn die Temperaturänderung eine Aufheizung oder eine Abkühlung umfasst. Eine Aufheizung, also Erwärmung, ist durch den Betrieb des Heizers möglich, während eine Abkühlung durch die Ausschaltung des Heizers möglich ist, wenn dieser zuvor in Betrieb war.

Bei der Ermittlung handelt es sich bevorzugterweise um eine Messung.

Darüber hinaus ist es bevorzugt, wenn das Verfahren vor einer Ermittlung der Wasserstoffkonzentration eines Gases durchgeführt wird. Hierdurch kann die Funktionsfähigkeit des Sensors vor dessen Nutzung überprüft werden.

Darüber hinaus ist es bevorzugt, wenn die Ermittlung des Diagnosewerts in Reaktion auf die Temperaturänderung im Messraum erfolgt.

Darüber hinaus ist es bevorzugt, wenn die Feststellung der Diagnose in Reaktion auf die Ermittlung des Diagnosewerts erfolgt.

Ein bevorzugtes Ausführungsbeispiel ist dadurch gekennzeichnet, dass die Temperaturänderung einen Betrieb des Heizers mit einer konstanten Heizleistung, eine Aufheizung mittels des Heizers durch eine Erhöhung der Heizleistung, eine Abkühlung aufgrund einer Deaktivierung und/oder einer Senkung der Heizleistung des Heizers umfasst.

Ein weiteres bevorzugtes Ausführungsbeispiel ist dadurch gekennzeichnet, dass der ermittelte Diagnosewert eine Temperatur, eine Temperaturänderungsgeschwindigkeit, einen Temperaturänderungsverlauf und/oder einen Zeitwert umfasst. Die Temperatur, insbesondere die Temperatur im Messraum, kann beispielsweise nach einem bestimmten Zeitabschnitt ermittelt oder gemessen werden. Unter der Temperaturänderungsgeschwindigkeit ist die Temperaturänderung pro Zeiteinheit zu verstehen. Unter dem Temperaturverlauf ist der Verlauf der Temperatur während eines Zeitabschnitts zu verstehen. Wenn lediglich eine Temperatur als Diagnosewert verwendet wird, ist dies besonders schnell, während die Temperaturänderungsgeschwindigkeit oder der Temperaturänderungsverlauf besonders genaue Rückschlüsse zulassen. Bei dem Zeitwert kann beispielsweise die verstrichene Zeit, die notwendig ist, von einer Ausgangstemperatur zu einer Zieltemperatur zu gelangen, verwendet werden. Wenn die Membran oder die Messraumöffnung durch einen Fremdkörper oder eine Flüssigkeit blockiert ist, heizt sich der Messraum schneller, also in kürzerer Zeit auf als bei freier Membran und freier Messraumöffnung.

Bei den zuvor genannten, unterschiedlich möglichen Diagnosewerten handelt es sich bevorzugterweise um Relativ- oder Absolutwerte.

Vorzugsweise wird vor Start der Verfahrens oder vor Beginn der Temperaturänderung die Anfangstemperatur im Messraum ermittelt und besonders bevorzugte bei der Feststellung der Diagnose oder bei der Ermittlung des Diagnosewerts berücksichtigt.

Ein weiteres bevorzugtes Ausführungsbeispiel ist dadurch gekennzeichnet, dass Verfahren den Verfahrensschritt, Vergleich des ermittelten Diagnosewerts mit einem hinterlegten Diagnosewert, umfasst. Bei dem hinterlegten Diagnosewert handelt es sich beispielsweise um einen zuvor experimentell ermittelten Diagnose- oder Vergleichswert. Es kann sich alternativ um einen zuvor mittels des erfindungsgemäßen Verfahrens ermittelten Diagnose- oder Vergleichswert handeln. Mit anderen Worten handelt es sich um einen historischen, zeitlich zurückliegenden, ermittelten Diagnose- oder Vergleichswert, der als hinterlegter Diagnose- oder Vergleichswert genutzt wird. Vorzugsweise erfolgt der Vergleich nach der Ermittlung und/ vor der Feststellung der Diagnose.

Ein weiteres bevorzugtes Ausführungsbeispiel ist dadurch gekennzeichnet, dass der hinterlegte Diagnosewert vom gleichen Typ ist, wie der ermittelte Diagnosewert. Beispielsweise wird ein ermittelter Temperaturänderungsverlauf mit einem hinterlegten Temperaturänderungsverlauf oder ein ermittelter Zeitwert mit einem hinterlegten Zeitwert verglichen.

Ein weiteres bevorzugtes Ausführungsbeispiel ist dadurch gekennzeichnet, dass bei Erreichung, Unterschreitung und/oder Überschreitung des hinterlegten Diagnosewerts mit dem ermittelten Diagnosewert eine Diagnose festgestellt wird. Durch diese Varianten kann auf verschiedene Einsatzbedingungen des Sensors eingegangen werden.

Ein weiteres bevorzugtes Ausführungsbeispiel ist dadurch gekennzeichnet, dass die Termperaturänderung im Messraum und die Ermittlung des Diagnosewerts zeitgleich, zeitversetzt oder direkt hintereinander durchgeführt werden. Hierdurch kann der gegenseitige Einfluss von Temperaturänderung und Ermittlung verändert werden.

Ein weiteres bevorzugtes Ausführungsbeispiel ist dadurch gekennzeichnet, dass die Termperaturänderung im Messraum und die Ermittlung des Diagnosewerts zeitgleich, zeitversetzt oder direkt hintereinander gestartet und/oder beendet werden. Auch hierdurch kann der gegenseitige Einfluss von Temperaturänderung und Ermittlung verändert werden.

Ein weiteres bevorzugtes Ausführungsbeispiel ist dadurch gekennzeichnet, dass die Termperaturänderung im Messraum und/oder die Ermittlung des Diagnosewerts bei Unterschreitung oder Überschreitung einer bestimmten Temperatur im Messraum gestartet wird, und/oder dass die Termperaturänderung im Messraum und/oder die Ermittlung des Diagnosewerts bei Unterschreitung oder Überschreitung einer bestimmten Temperatur im Messraum beendet wird.

Ein weiteres bevorzugtes Ausführungsbeispiel ist dadurch gekennzeichnet, dass die Feststellung der Diagnose die Feststellung einer Verstopfung der Membran mit einem Fremdkörper oder einer Flüssigkeit umfasst. Bei diesen beiden Feststellungen handelt es sich um zwei typische Ursachen, die Einfluss auf die zu ermittelten Diagnosewerte haben. Daher kann in Abhängigkeit des Diagnosewerts auf einen dieser beiden Zustände geschlossen werden, insbesondere wenn der ermittelte Diagnosewert mit einem hinterlegten Diagnosewert verglichen wird, wobei der hinterlegte Diagnosewert, einem der beiden Zustände entspricht.

Darüber hinaus ist es vorteilhaft, wenn bei Feststellung einer Verstopfung der Membran mit einem Fremdkörper oder einer Flüssigkeit eine Messung mittels des Wasserstoffsensor verweigert wird oder nicht möglich ist. Vorzugsweise ist dies nicht möglich, solange durch das erfindungsmäße Verfahren feststellt wird, dass die Membran mit einem Fremdkörper oder einer Flüssigkeit verstopft ist. Hierfür ist es zweckmäßig das Verfahren mehrmals, insbesondere zeitlich beabstandet, zu wiederholen.

Alternativ ist es denkbar Messwerte, die Konzentration von Wasserstoff im zu messenden Gas betreffend, die mittels der Wasserstoffsensors nach der Feststellung einer Verstopfung der Membran mit einem Fremdkörper oder einer Flüssigkeit, ermittelt werden als unzulässig oder fehlerhaft zu markieren, zu kennzeichnen oder abzuspeichern. Auf diese Weise kann verhindert werden, dass die Messwerte zur Regelung oder Steuerung eines Systems, insbesondere eines Brennstoffzellensystems genutzt werden.

Alternativ oder zusätzlich zu einer der beiden vorangegangenen Ausführungen ist es denkbar, dass die Heizleistung des Heizers erhöht wird, um den Fremdkörper oder die Flüssigkeit zu entfernen. Alternativ oder zusätzlich hierzu ist es denkbar, die Strömungsgeschwindigkeit des zu messenden Gases kurzzeitig zu erhöhen, um den Fremdkörper oder die Flüssigkeit zu entfernen.

Darüber hinaus ist es besonders bevorzugt, wenn im Fall, dass die Feststellung einer Diagnose erfolgt, wonach die Membran oder die Messraumöffnung frei von einer Flüssigkeit oder einem Fremdkörper sind, das Verfahren beendet wird.

Ein weiteres bevorzugtes Ausführungsbeispiel ist dadurch gekennzeichnet, dass es sich bei dem Verfahren um ein computerimplementiertes Verfahren handelt.

Die Aufgabe bezüglich des Wasserstoffsensors wird dadurch gelöst, dass ein Wasserstoffsensor, der einen Messraum, einen Heizer und einen Temperaturfühler, wobei der Messraum mittels einer Messraumöffnung fluidisch mit der Umgebung des Wasserstoffsensors verbunden ist,
wobei die Messraumöffnung mittels einer Membran verschlossen ist,
wobei die Membran gasdurchlässig und flüssigkeitsundurchlässig ist, und Mitteln umfasst, die so angepasst sind, dass sie die Schritte des erfindungsgemäßen Verfahrens ausführen, bereitgestellt wird.

Auch ist es zu bevorzugen, wenn der Wasserstoffsensor ferner mit einem Steuergerät gekoppelt ist, welches insbesondere die hinterlegten Diagnosewerte umfasst. Auch ist es zweckmäßig, wenn das Steuergerät eine Auswerteeinheit umfasst, welche die Feststellung der Diagnose und/oder den Vergleich des ermittelten Diagnosewerts mit einem hinterlegten Diagnosewert durchführt.

Besonders vorteilhaft ist es, wenn der Heizer und der Temperaturfühler im Messraum angeordnet sind. Dies ermöglicht eine schnellere Aufheizung und eine genauere Messung.

Auch ist es vorteilhaft, wenn es sich bei dem Wasserstoffsensor um einen Sensor zu Ermittlung einer Wasserstoffkonzentration in einem Gas handelt. Solche Sensoren werden in Abgaskanälen von Brennstoffzellensystemen eingesetzt.

Auch ist es zu bevorzugen, wenn der Wasserstoffsensor ein Sensorgehäuse umfasst, das insbesondere den Messraum begrenzt.

Die Aufgabe bezüglich des Computerprogramms wird dadurch gelöst, dass ein Computerprogramm, umfassend Befehle, die bewirken, dass der erfindungsgemäße Wasserstoffsensor die Verfahrensschritte des erfindungsgemäßen Verfahrens ausführt.

Die Aufgabe bezüglich des computerlesbaren Mediums wird dadurch gelöst, dass ein computerlesbares Medium bereitgestellt wird, auf das erfindergemäße Computerprogramm gespeichert ist.

Auch ist es zu bevorzugen, wenn das computerlesbare Medium Teil des Steuergeräts ist, beispielsweise als permanenter oder temporärer Speicher.

Vorteilhafte Weiterbildungen der vorliegenden Erfindung sind in den Unteransprüchen und in der nachfolgenden Figurenbeschreibung beschrieben.

### Kurze Beschreibung der Zeichnungen

Im Folgenden wird die Erfindung anhand von Ausführungsbeispielen unter Bezugnahme auf die Zeichnungen detailliert erläutert. In den Zeichnungen zeigen:
- Fig. 1: ein Kraftfahrzeug,
- Fig. 2: einen Kanal mit einem Wasserstoffsensor, und
- Fig. 3: ein Ablaufdiagramm des Verfahrens.

### Bevorzugte Ausführung der Erfindung

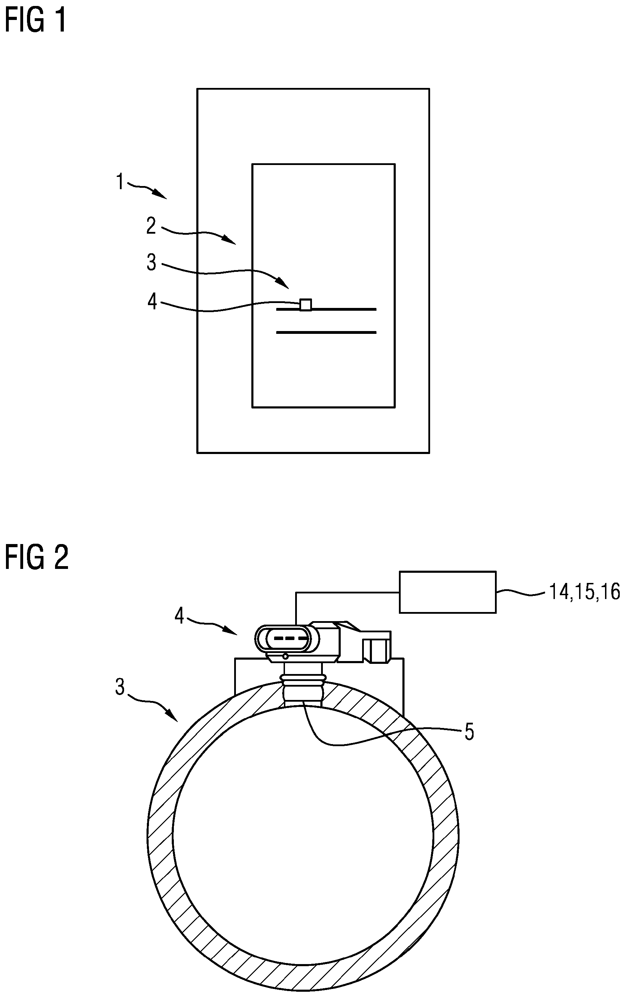

Die Figur 1 zeigt ein Kraftfahrzeug 1 mit Elektroantrieb und einem Brennstoffzellensystem 2. Das Brennstoffzellensystem 2 umfasst einen Kanal 3, der als Abgaskanal des Brennstoffzellensystems 2 ausgebildet ist und in dem die Wasserstoffkonzentration mittels eines Wasserstoffsensors 4 ermittelt werden kann.

Die Figur 2 zeigt den Kanal 3 und den Wasserstoffsensor 4 aus Figur 1. Der Wasserstoffsensor 4 ist in einer Öffnung der Kanalwand des Kanals 3 befestigt und umfasst einen Messraum, der fluidisch mittels einer Messraumöffnung mit dem Kanal 3 verbunden ist. Die Messraumöffnung ist mittels einer Membran 5, die gasdurchlässig und flüssigkeitsundurchlässig ist, verschlossen. Im Messraum des Wasserstoffsensors 4 ist ein Heizer und ein Temperaturfühler angeordnet. Mittels der hohen Wärmekapazität von Wasserstoff im Vergleich zu den anderen Bestandteilen des zu messenden Gases lässt sich mithilfe des Heizers und des Temperaturfühler die Konzentration von Wasserstoff in dem zu messenden Gas ermitteln. Mit anderen Worten handelt es sich bei dem Wasserstoffsensor 4 um einen thermischen Gassensor. Der Wasserstoffsensor 4 ist mit einem Steuergerät 14 gekoppelt, dass ein computerlesbares Medium 15 in Form eines permanenten Speichers integriert ist. Auf dem computerlesbaren Medium 15 ist ein Computerprogramm 16, umfassend Befehle, die bewirken, dass der Wasserstoffsensor 4 und/oder eine in das Steuergerät 14 integrierte Auswerteeinheit die Verfahrensschritte des erfindungsgemäßen Verfahrens ausführt, abgespeichert.

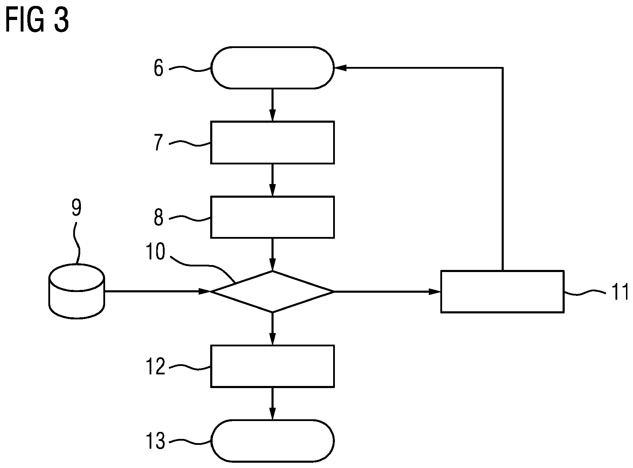

Die Figur 3 zeigt ein Ablaufdiagramm des erfindungsgemäßen Verfahrens, wie es in den Vorrichtungen der Figuren 1 und 2 zur Anwendung kommt. Zunächst erfolgt der Start 6 des Verfahrens, bevor eine Temperaturänderung 7 im Messraum des Wasserstoffsensors mittels des Heizer Messraum durchgeführt wird. Bei der Temperaturänderung 7 handelt es sich um einen Heizvorgang, der die Temperatur im Messraum erhöht. Zeitgleich mit der Temperaturänderungen 7 erfolgt eine Ermittlung 8 eines Diagnosewerts mittels des Temperaturfühlers. Bei dem ermittelten Diagnosewert handelt es sich um die Zeit, die verstreicht, bis eine gewisse Temperatur innerhalb des Messraums erreicht ist. Daraufhin erfolgt ein Vergleich 10 dieses Diagnosewerts wird mit einem hinterlegten Diagnosewert 9. Wenn der ermittelte Diagnosewert unter dem hinterlegten Diagnosewert 9 liegt, bedeutet es, dass sich die Temperatur im Messraum schneller erhöht hat als üblich, da der hinterlegte Diagnosewert 9 einem Zeitwert entspricht, der ermittelbar ist, wenn die Membran des Wasserstoffsensors weder durch eine Flüssigkeit noch durch einen Fremdkörper blockiert ist. In dem Fall, dass der ermittelte Diagnosewert unter dem hinterlegten Diagnosewert 9 liegt, erfolgt daher die Feststellung 11, dass die Membran oder die Messraumöffnung durch einen Fremdkörper oder eine Flüssigkeit blockiert ist. Daraufhin ist es möglich, zu einem späteren Zeitpunkt das Verfahren neu zu starten, um festzustellen, ob diese Diagnose weiterhin besteht. Darüber hinaus ist es denkbar, den Heizer weiterhin zu betreiben, und die Flüssigkeit oder den Fremdkörper zu entfernen. Ferner ist es denkbar ein Reinigungsverfahren zur Reinigung der Membran oder der Messraumöffnung zu initialisieren. Auch ist es denkbar, dass Messwerte, die Konzentration von Wasserstoff im zu messenden Gas betreffend, als fehlerhaft abgespeichert und daher nicht für weitere Verfahren verwendet werden. Im Fall, dass der ermittelte Diagnosewert mindestens dem hinterlegten Diagnosewert 9 entspricht, erfolgt die Feststellung 12, wonach die Membran oder die Messraumöffnung frei von einer Flüssigkeit oder einem Fremdkörper sind. Daraufhin erfolgt die Beendigung 13 des Verfahrens, wodurch im Anschluss ein Messverfahren zur Ermittlung der Konzentration von Wasserstoff im zu messenden Gas initialisiert werden kann.

Die unterschiedlichen Merkmale der einzelnen Ausführungsbeispiele können auch untereinander kombiniert werden.

Die Ausführungsbeispiele der Figuren 1 bis 3 weisen insbesondere keinen beschränkenden Charakter auf und dienen der Verdeutlichung des Erfindungsgedankens.

### Bezugszeichenliste

- 1: Kraftfahrzeug
- 2: Brennstoffzellensystem
- 3: Kanal
- 4: Wasserstoffsensor
- 5: Membran
- 6: Start
- 7: Temperaturänderung
- 8: Ermittlung
- 9: hinterlegter Diagnosewert
- 10: Vergleich
- 11: Feststellung
- 12: Feststellung
- 13: Beendigung
- 14: Steuergerät
- 15: computerlesbares Medium
- 16: Computerprogramm

## Patentansprüche

1. Verfahren zur Diagnose eines Wasserstoffsensors (4),
wobei der Wasserstoffsensor (4) einen Messraum, einen Heizer und einen Temperaturfühler umfasst,
wobei der Messraum mittels einer Messraumöffnung fluidisch mit der Umgebung des Wasserstoffsensors verbunden ist,
**dadurch gekennzeichnet, dass** die Messraumöffnung mittels einer Membran (5) verschlossen ist, wobei die Membran (5) gasdurchlässig und flüssigkeitsundurchlässig ist, umfassend die Schritte:
- Temperaturänderung (7) im Messraums mittels des Heizers,
- Ermittlung (8) eines Diagnosewerts im Messraum mittels des Temperaturfühlers,
- Feststellung (11, 12) einer Diagnose in Abhängigkeit des Diagnosewerts.

2. Verfahren nach Anspruch 1, **dadurch gekennzeichnet, dass** die Temperaturänderung (7) einen Betrieb des Heizers mit einer konstanten Heizleistung, eine Aufheizung mittels des Heizers durch eine Erhöhung der Heizleistung, eine Abkühlung aufgrund einer Deaktivierung und/oder einer Senkung der Heizleistung des Heizers umfasst.

3. Verfahren nach Anspruch 1 oder 2, **dadurch gekennzeichnet, dass** der ermittelte Diagnosewert eine Temperatur, eine Temperaturänderungsgeschwindigkeit, einen Temperaturänderungsverlauf und/oder einen Zeitwert umfasst.

4. Verfahren nach einem der vorhergehenden Ansprüche, **dadurch gekennzeichnet, dass** Verfahren den Verfahrensschritt, Vergleich (10) des ermittelten Diagnosewerts mit einem hinterlegten Diagnosewert (9), umfasst.

5. Verfahren nach Anspruch 4, **dadurch gekennzeichnet, dass** der hinterlegte Diagnosewert (9) vom gleichen Typ ist, wie der ermittelte Diagnosewert.

6. Verfahren nach einem der vorhergehenden Ansprüche, **dadurch gekennzeichnet, dass** bei Erreichung, Unterschreitung und/oder Überschreitung des hinterlegten Diagnosewerts (9) mit dem ermittelten Diagnosewert eine Diagnose festgestellt wird.

7. Verfahren nach einem der vorhergehenden Ansprüche, **dadurch gekennzeichnet, dass** die Termperaturänderung (7) im Messraum und die Ermittlung (8) des Diagnosewerts zeitgleich, zeitversetzt oder direkt hintereinander durchgeführt werden.

8. Verfahren nach einem der vorhergehenden Ansprüche, **dadurch gekennzeichnet, dass** die Termperaturänderung (7) im Messraum und die Ermittlung (8) des Diagnosewerts zeitgleich, zeitversetzt oder direkt hintereinander gestartet und/oder beendet werden.

9. Verfahren nach einem der vorhergehenden Ansprüche, **dadurch gekennzeichnet, dass** die Termperaturänderung (7) im Messraum und/oder die Ermittlung (8) des Diagnosewerts bei Unterschreitung oder Überschreitung einer bestimmten Temperatur im Messraum gestartet wird, und/oder dass die Termperaturänderung (7) im Messraum und/oder die Ermittlung (8) des Diagnosewerts bei Unterschreitung oder Überschreitung einer bestimmten Temperatur im Messraum beendet wird.

10. Verfahren nach einem der vorhergehenden Ansprüche, **dadurch gekennzeichnet, dass** die Feststellung (11, 12) der Diagnose die Feststellung einer Verstopfung der Membran mit einem Fremdkörper oder einer Flüssigkeit umfasst.

11. Verfahren nach einem der vorhergehenden Ansprüche, **dadurch gekennzeichnet, dass** es sich bei dem Verfahren um ein computerimplementiertes Verfahren handelt.

12. Wasserstoffsensor (4), der einen Messraum, einen Heizer und einen Temperaturfühler,
wobei der Messraum mittels einer Messraumöffnung fluidisch mit der Umgebung des Wasserstoffsensors (4) verbunden ist,
**dadurch gekennzeichnet, dass** die Messraumöffnung mittels einer Membran (5) verschlossen ist, wobei die Membran (5) gasdurchlässig und flüssigkeitsundurchlässig ist, und Mitteln umfasst, die so angepasst sind, dass sie die Schritte (7, 8, 10, 11, 12) des Verfahrens nach einem der vorhergehenden Ansprüche ausführen.

13. Computerprogramm (16), umfassend Befehle, die bewirken, dass die Vorrichtung des Anspruchs 12 die Verfahrensschritte (7, 8, 10, 11, 12) nach einem der Ansprüche 1 bis 11 ausführt.

14. Computerlesbares Medium (15), auf dem das Computerprogramm (16) nach Anspruch 13 gespeichert ist.

## Claims

1. Method of diagnosing a hydrogen sensor (4), wherein the hydrogen sensor (4) comprises a measurement space, a heater and a temperature sensor,
wherein the measurement space is fluidically connected to the environment of the hydrogen sensor by means of a measurement space opening,
**characterized in that**
the measurement space opening is closed by means of a membrane (5),
wherein the membrane (5) is gas-permeable and liquid-impermeable,
comprising the steps of:
- changing the temperature (7) in the measurement space by means of the heater,
- ascertaining (8) a diagnosis value in the measurement space by means of the temperature sensor,
- determining (11, 12) a diagnosis depending on the diagnosis value.

2. Method according to Claim 1, **characterized in that** the change in temperature (7) comprises operation of the heater with a constant heating output, heating by means of the heater by an increase in the heating output, cooling owing to deactivation and/or lowering of the heating output of the heater.

3. Method according to Claim 1 or 2, **characterized in that** the ascertained diagnosis value comprises a temperature, a rate of temperature change, a progression of temperature change and/or a time value.

4. Method according to any of the preceding claims, **characterized in that** the method comprises the method step of comparing (10) the ascertained diagnosis value with a recorded diagnosis value (9).

5. Method according to Claim 4, **characterized in that** the recorded diagnosis value (9) is of the same type as the ascertained diagnosis value.

6. Method according to any of the preceding claims, **characterized in that** a diagnosis is determined when the ascertained diagnosis value attains, goes below and/or goes above the recorded diagnosis value (9).

7. Method according to any of the preceding claims, **characterized in that** the change in temperature (7) in the measurement space and the ascertaining (8) of the diagnosis value are conducted simultaneously, after a time delay or in direct succession.

8. Method according to any of the preceding claims, **characterized in that** the change in temperature (7) in the measurement space and the ascertaining (8) of the diagnosis value are started and/or ended simultaneously, after a time delay or in direct succession.

9. Method according to any of the preceding claims, **characterized in that** the change in temperature (7) in the measurement space and/or the ascertaining (8) of the diagnosis value is started when the temperature in the measurement space goes below or above a particular temperature, and/or **in that** the change in temperature (7) in the measurement space and/or the ascertaining (8) of the diagnosis value is ended when the temperature in the measurement space goes below or above a particular temperature.

10. Method according to any of the preceding claims, **characterized in that** the determining (11, 12) of the diagnosis comprises the determining of a blockage of the membrane with a foreign body or a liquid.

11. Method according to any of the preceding claims, **characterized in that** the method is a computer-implemented method.

12. Hydrogen sensor (4) comprising a measurement space, a heater and a temperature sensor,
wherein the measurement space is fluidically connected to the environment of the hydrogen sensor (4) by means of a measurement space opening,
**characterized in that**
the measurement space opening is closed by means of a membrane (5),
wherein the membrane (5) is gas-permeable and liquid-impermeable, and comprising means adapted to execute the steps (7, 8, 10, 11, 12) of the method according to any of the preceding claims.

13. Computer program (16) comprising commands that cause the device of Claim 12 to execute the method steps (7, 8, 10, 11, 12) according to any of Claims 1 to 11.

14. Computer-readable medium (15) on which the computer program (16) according to Claim 13 is stored.

## Revendications

1. Procédé de diagnostic d'un capteur d'hydrogène (4), le capteur d'hydrogène (4) comprenant un espace de mesure, un dispositif de chauffage et un capteur de température,
l'espace de mesure étant relié fluidiquement à l'environnement du capteur d'hydrogène au moyen d'une ouverture d'espace de mesure,
**caractérisé en ce que**
l'ouverture d'espace de mesure est fermée au moyen d'une membrane (5),
la membrane (5) étant perméable aux gaz et imperméable aux liquides,
comprenant les étapes suivantes :
- modification de la température (7) dans l'espace de mesure au moyen du dispositif de chauffage,
- détermination (8) d'une valeur de diagnostic dans l'espace de mesure au moyen du capteur de température,
- établissement (11, 12) d'un diagnostic en fonction de la valeur de diagnostic.

2. Procédé selon la revendication 1, **caractérisé en ce que** la modification de température (7) comprend un fonctionnement du dispositif de chauffage avec une puissance de chauffage constante, un chauffage au moyen du dispositif de chauffage par une augmentation de la puissance de chauffage, le refroidissement dû à une désactivation et/ou à une réduction de la puissance de chauffage du dispositif de chauffage.

3. Procédé selon la revendication 1 ou 2, **caractérisé en ce que** la valeur de diagnostic déterminée comprend une température, une vitesse de modification de température, un profil de modification de température et/ou une valeur de temps.

4. Procédé selon l'une quelconque des revendications précédentes, **caractérisé en ce que** le procédé comprend l'étape de procédé de comparaison (10) de la valeur de diagnostic déterminée avec une valeur de diagnostic mémorisée (9).

5. Procédé selon la revendication 4, **caractérisé en ce que** la valeur de diagnostic mémorisée (9) est du même type que la valeur de diagnostic déterminée.

6. Procédé selon l'une quelconque des revendications précédentes, **caractérisé en ce qu'**un diagnostic est établi en cas d'atteinte, de franchissement vers le bas et/ou de franchissement vers le haut de la valeur de diagnostic mémorisée (9) par la valeur de diagnostic déterminée.

7. Procédé selon l'une quelconque des revendications précédentes, **caractérisé en ce que** la modification de température (7) dans l'espace de mesure et la détermination (8) de la valeur de diagnostic sont effectuées simultanément, de manière décalée dans le temps ou directement l'une après l'autre.

8. Procédé selon l'une quelconque des revendications précédentes, **caractérisé en ce que** la modification de température (7) dans l'espace de mesure et la détermination (8) de la valeur de diagnostic sont démarrées et/ou terminées simultanément, de manière décalée dans le temps ou directement l'une après l'autre.

9. Procédé selon l'une quelconque des revendications précédentes, **caractérisé en ce que** la modification de température (7) dans l'espace de mesure et/ou la détermination (8) de la valeur de diagnostic sont démarrées en cas de franchissement vers le bas ou vers le haut d'une température déterminée dans l'espace de mesure, et/ou **en ce que** la modification de température (7) dans l'espace de mesure et/ou la détermination (8) de la valeur diagnostique sont terminées en cas de franchissement vers le bas ou vers le haut d'une température déterminée dans l'espace de mesure.

10. Procédé selon l'une quelconque des revendications précédentes, **caractérisé en ce que** l'établissement (11, 12) du diagnostic comprend l'établissement d'une obstruction de la membrane par un corps étranger ou un liquide.

11. Procédé selon l'une quelconque des revendications précédentes, **caractérisé en ce qu'**il s'agit d'un procédé mis en œuvre par ordinateur.

12. Capteur d'hydrogène (4) comprenant un espace de mesure, un dispositif de chauffage et un capteur de température,
l'espace de mesure étant relié fluidiquement à l'environnement du capteur d'hydrogène (4) au moyen d'une ouverture d'espace de mesure,
**caractérisé en ce que**
l'ouverture d'espace de mesure est fermée au moyen d'une membrane (5),
la membrane (5) étant perméable aux gaz et imperméable aux liquides, et comportant des moyens adaptés pour exécuter les étapes (7, 8, 10, 11, 12) du procédé selon l'une quelconque des revendications précédentes.

13. Programme informatique (16) comprenant des instructions qui amènent le dispositif de la revendication 12 à exécuter les étapes de procédé (7, 8, 10, 11, 12) selon l'une quelconque des revendications 1 à 11.

14. Support lisible par ordinateur (15) sur lequel est mémorisé le programme informatique (16) selon la revendication 13.
